# FASCICULE DE BREVET EUROPEEN

(11) **EP 2 755 649 B1**
(45) Date de publication et mention de la délivrance du brevet: **14.12.2016**
(21) Numéro de dépôt: 12759430.7
(22) Date de dépôt: 13.09.2012
(51) Int. Cl.: A61K 31/38, A61K 31/39, A61K 9/00, A61P 9/00

(54) **UTILISATION DE LA 3-(R)-[3-(2-METHOXYPHENYLTHIO)-2-(S)-METHYL-PROPYL]AMINO-3,4-DIHYDRO-2H-1,5-BENZOXATHIEPINE POUR LA PREVENTION ET/OU LE TRAITEMENT DES EFFETS CARDIOTOXIQUES DUS A LA CHIMIOTHERAPIE ET/OU LES RAYONNEMENTS**
VERWENDUNG VON 3-(R)-[3-(2-METHOXYPHENYLTHIO-)2-(S)-METHYLPROPYL]AMINO-3,4-DIHYDRO-2H-1,5-BENZOXATHIEPIN ZUR PRÄVENTION UND/ODER BEHANDLUNG VON DURCH CHEMOTHERAPIE UND/ODER BESTRAHLUNG VERURSACHTEN KARDIOTOXISCHEN WIRKUNGEN
USE OF 3-(R)-[3-(2-METHOXYPHENYLTHIO)-2-(S)-METHYLPROPYL]AMINO-3,4-DIHYDRO- 2H-1,5-BENZOXATHIEPINE FOR PREVENTING AND/OR TREATING CARDIOTOXIC EFFECTS CAUSED BY CHEMOTHERAPY AND/OR RADIATION

(30) Priorité: 13.09.2011 FR 1158155
(43) Date de publication de la demande: 23.07.2014
(73) Titulaire: Pierre Fabre Médicament, 92100 Boulogne-Billancourt (FR)
(72) Inventeur: VACHER, Bernard, F-81100 Castres (FR); LE GRAND, Bruno, F-81220 Teyssode (FR)
(74) Mandataire: Regimbeau
(86) Numéro de dépôt international: PCT/EP2012/067980
(87) Numéro de publication internationale: WO 2013/037905

(56) Documents cités:
- WO-A1-2011/003129
- FR-A1- 2 822 467
- LETIENNE R ET AL: "Myocardial protection by F 15845, a persistent sodium current blocker, in an ischemia-reperfusion model in the pig", EUROPEAN JOURNAL OF PHARMACOLOGY, ELSEVIER SCIENCE, NL, vol. 624, no. 1-3, 10 décembre 2009 (2009-12-10), pages 16-22, XP026747490, ISSN: 0014-2999, DOI: 10.1016/J.EJPHAR.2009.09.032 [extrait le 2009-09-22]
- SAINT DAVID A: "Persistent (current) in the face of adversity ... a new class of cardiac anti-ischaemic compounds on the horizon?", BRITISH JOURNAL OF PHARMACOLOGY JAN 2009 LNKD- PUBMED:19133984, vol. 156, no. 2, janvier 2009 (2009-01), pages 211-213, XP002672467, ISSN: 1476-5381
- GILLET L ET AL: "Beneficial effects of omega-3 long-chain fatty acids in breast cancer and cardiovascular diseases: voltage-gated sodium channels as a common feature?", BIOCHIMIE, MASSON, PARIS, FR, vol. 93, no. 1, 1 janvier 2011 (2011-01-01), pages 4-6, XP027564848, ISSN: 0300-9084 [extrait le 2010-02-16]
- BELARDINELLI L ET AL: "Inhibition of the late sodium current as a potential cardioprotective principle: effects of the late sodium current inhibitor ranolazine.", HEART (BRITISH CARDIAC SOCIETY) JUL 2006 LNKD- PUBMED:16775092, vol. 92 Suppl 4, juillet 2006 (2006-07), pages IV6-IV14, XP002672468, ISSN: 1468-201X
- EWER MICHAEL S ET AL: "Cardiotoxicity of anticancer treatments: what the cardiologist needs to know.", NATURE REVIEWS. CARDIOLOGY OCT 2010 LNKD- PUBMED:20842180, vol. 7, no. 10, octobre 2010 (2010-10), pages 564-575, XP009157868, ISSN: 1759-5010
- SINGAL PAWAN K ET AL: "Doxorubicin-induced cardiomyopathy", NEW ENGLAND JOURNAL OF MEDICINE, vol. 339, no. 13, 24 septembre 1998 (1998-09-24), pages 900-905, XP002672469, ISSN: 0028-4793

## Description

L'invention concerne la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables pour son utilisation dans la prévention et/ou le traitement des effets cardiotoxiques dus à la chimiothérapie et ou les rayonnements anticancéreux.

La 3-(R)-[3-(2-méthoxyphénylthio)-2-(S)-méthyl-propyl]amino-3,4-dihydro-2H-1,5-benzoxathiépine représenté par la formule : 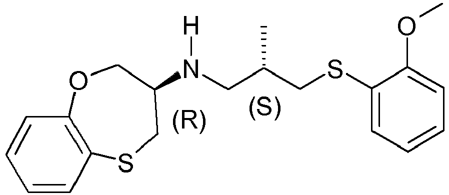 ses sels pharmaceutiquement acceptables ainsi que son utilisation dans le traitement de l'angor, de l'insuffisance cardiaque, de l'infarctus du myocarde et des troubles du rythme cardiaque sont décrit dans le brevet WO 02/081464.

Le cancer peut être défini de façon très large comme une maladie liée à la prolifération et la diffusion incontrôlée de cellules de l'organisme devenues anormales. C'est l'une des premières causes de mortalité dans les pays développés et le nombre de nouveaux cas est en constante augmentation. Cependant, grâce entre autres aux progrès des traitements anticancéreux le taux de mortalité par cancer a significativement diminué. Les traitements anticancéreux incluent, suivant le type et le degré d'évolution de la maladie, la chirurgie, la radiothérapie et la chimiothérapie. Dans la plupart des cas, une combinaison de deux ou trois approches est nécessaire.

La radiothérapie est une méthode de traitement locorégional des cancers, utilisant des radiations ionisantes pour détruire les cellules cancéreuses tout en épargnant autant que possible les tissus sains voisins. On entend par « radiation ionisante », les particules chargées ou non chargées ou les rayonnements énergétiques capables de transmettre à la cellule irradiée leur énergie. Les radiations ionisantes altèrent la structure des macromolécules et perturbent les principales fonctions de la vie cellulaire.

La chimiothérapie consiste en l'utilisation de substances synthétiques ou naturelles susceptibles de tuer ou de limiter la prolifération des cellules cancéreuses. Par « chimiothérapie conventionnelle » on entend une chimiothérapie basée sur l'utilisation d'agents cytotoxiques. Par « chimiothérapie ciblée » on entend l'utilisation d'agents actifs sur une cible biologique précise intervenant dans le processus de cancérogénèse.

Les chimiothérapies anticancéreuses qu'elles soient ciblées ou conventionnelles interfèrent avec des mécanismes présents dans la plupart des types cellulaires et impliqués dans la croissance, la survie et/ou la multiplication des cellules. Ils compromettent de ce fait l'homéostasie cellulaire non seulement dans les tissus cancéreux mais aussi dans les tissus non-cancéreux de l'organisme. Ce manque de sélectivité d'action vis à vis des cellules cancéreuses a pour corollaire une importante toxicité.

Le coeur est particulièrement sensible à l'action systémique des substances chimiothérapeutiques et des radiations ionisantes. En effet, comme le tissu cardiaque ne se renouvelle pas ou très peu, les cellules musculaires cardiaques (cardiomyocytes) sont naturellement protégées contre l'apoptose. Or, les mécanismes mis en oeuvre par les principaux médicaments anticancéreux ainsi que par les radiations ionisantes reposent précisément sur la réactivation/stimulation des voies pro-apoptotiques et/ou l'inhibition des voies anti-apoptotiques.

Ainsi, chez les patients guéris ou ceux en rémission prolongée, il ressort que la mortalité par maladies cardiovasculaires due aux traitements anticancéreux dépasse celle associée à une récidive ou à l'apparition d'un nouveau cancer.

Les médicaments anticancéreux peuvent être classés schématiquement en plusieurs catégories selon leurs cibles cellulaires et/ou leur mécanisme d'action.
- les agents cytotoxiques :
   o les agents alkylants et apparentés : ils possèdent des fonctions capables de se lier à de nombreux substrats nucléophiles. Ces groupements réactifs se fixent notamment sur l'ADN et forment des ponts intra ou intercaténaires bloquant ainsi sa réplication. On peut citer par exemple comme agents alkylants l'altéramine, le busulfan, le carboplatine, la carmustine, le chlorambucil, la chlorméthine, le cisplatine, le cyclophosphamide, la dacarbazine, l'estramustine, la fotémustine, l'ifosfamide, la lomustine, la méchloréthamine, le melphalan, la mitomycine C, l'oxaliplatine, le pipobroman, le procarbazine, la streptozocine, le témozolomide, le thiotépa, la trabectédine, le trophosphamide, l'uramustine.
   o Les antimétabolites : ce sont des analogues structuraux des métabolites naturels, ils interfèrent avec la synthèse des acides nucléiques et sont donc principalement actifs pendant la phase S du cycle cellulaire. Ces médicaments agissent en inhibant des voies enzymatiques impliquées dans la synthèse *de novo* des bases puriques ou pyrimidiques ou en s'incorporant à leur place dans l'ADN. On peut citer par exemple comme antimétabolites l'azathioprine, la capécitabine, la cladribine, la cytarabine, la cytosine arabinoside, la floxuridine, la fludarabine, le 5- fluoro-uracile, la gemcitabine, la 6-mercapto-purine, le méthotrexate, le pemetrexed, la pentostatine, le raltitrexed, le tégafur, la thio-guanine.
   ∘ Les modificateurs de l'ADN : dans cette classe on trouve les inhibiteurs des topoisomérases qui sont des enzymes provoquant des coupures provisoires au niveau de l'ADN. Les topoisomérases I et II provoquent, respectivement, des cassures double-brin et simple-brin. Les inhibiteurs des topoisomérases se lient au complexe topoisomérase-ADN au stade de clivage et empêchent l'étape de religation. Les inhibiteurs des topoisomérases I comprennent l'irinotécan et le topotécan. Comme inhibiteur des topoisomérases II on peut citer l'étoposide, le mitopodoside, le téniposide. Parmi les modificateurs de l'ADN on trouve aussi les agents intercalants tels que les anthracyclines comme par exemple l'aclarubicine, la daunorubicine, la doxorubicine, l'épirubicine, l'idarubicine, la pirarubicine, la zorubicine et les anthracènediones comme par exemple la mitoxantrone. Ces molécules présentent une structure plane leur permettant de s'intercaler au sein de la double hélice d'ADN ce qui provoque sa déformation et bloque la transcription et la réplication. Ces dérivés possèdent de plus une activité sur les topoisomérases qu'ils vont inhiber.
      Les anthracyclines appartiennent aussi à la famille des antibiotiques antitumoraux qui elle même regroupe des composés tels que, par exemple, la bléomycine, la
      dactinomycine ou actinomycine D.
   ∘ Les dérivés tubulo-affins : deux groupes de molécules sont représentés dans cette classe. Le premier groupe concerne les vinca-alcaloïdes tels que la vinblastine, la vincaleucoblastine, la vincristine, la vindésine, la vinflunine, la vinorelbine et plus récemment l'eribulin, un analogue synthétique d'un produit naturel, l'halicondrine B, extrait d'éponges. Le second groupe comprend les taxanes e.g., le docétaxel, le paclitaxel et les épothilones tel que l'ixabepilone approuvée pour le traitement des cancers du sein résistants aux taxanes. Leur cible moléculaire commune est la tubuline cytoplasmique nécessaire à la construction du fuseau mitotique. Leurs effets sont surtout manifestes sur les cellules en division. Cependant, toute autre activité cellulaire reposant sur l'activité microtubulaire sera aussi bloquée.
- Les inhibiteurs de facteurs de croissances : les facteurs de croissance sont des polypeptides ou protéines qui stimulent la multiplication cellulaire des tissus. Ces facteurs de croissance sont reconnus par des récepteurs membranaires spécifiques. Le plus souvent il s'agit de récepteurs à tyrosine kinase. On distingue les récepteurs à une seule chaîne d'acides aminés avec par exemple la famille des récepteurs à l'EGF (epithelial growth factor) et les récepteurs à deux chaînes d'acides aminés avec par exemple les récepteurs du facteur de croissance vasculaire (VEGF) et dérivé des plaquettes (PDGF) tous deux très impliqués dans l'angiogénèse. On entend par angiogenèse, l'ensemble des processus qui aboutissent à la formation de nouveaux capillaires sanguins à partir du réseau vasculaire préexistant. Les inhibiteurs de l'angiogenèse sont aussi appelés médicaments anti-angiogéniques. Pour inhiber ces facteurs de croissances de façon spécifique, on va trouver deux classes de médicaments :
   ∘ Les anticorps monoclonaux: ce sont de grosses molécules actives par voie injectable. Ils bloquent soit le domaine extracellulaire du récepteur et provoquent son internalisation dans la cellule, soit se fixent sur le ligand endogène et empêchent sa reconnaissance par le récepteur. On peut citer par exemple l'alemtuzumab, le bevacizumab, le cetuximab, l'ibritumomab, le panitumumab, le pertuzumab, le rituximab, le trastuzumab.
   ∘ Les inhibiteurs de tyrosine kinase : ce sont des petites molécules généralement actives par voie orale et qui agissent sur le domaine intracellulaire du récepteur. Elles demeurent actives lorsqu'un ligand est fixé sur le récepteur. On peut citer par exemple le dasatinib, l'erlotinib, le gefitinib, l'imatinib, le nicotinib, le sunitinib, le sorafenib.
- L'hormonothérapie : la croissance des tumeurs dans certains cancers tels que ceux du sein, de l'endomètre, de la prostate, de la thyroïde dépend de la présence d'hormones telles que, par exemple, la 5-hydroxytestostérone, la progestérone, les oestrogènes. L'hormonothérapie utilise donc des inhibiteurs de la production de ces hormones ou de l'action de ces dernières. Dans cette classe on trouve les anti-aromatases, on peut citer par exemple l'anastrozole, le letrozole, l'exemestane, formestane ; les modulateurs sélectifs du récepteur des oestrogènes comme par exemple le tamoxifène, le torémifène ; les régulateurs négatifs du récepteur des oestrogènes comme par exemple le fulvestrant ; les anti-androgène avec par exemple le bicalutamide, le flutamide, le nilutamide ; les agonistes LH-RH comme par exemple la buséréline, la goséréline, la leuproréline, la nafaréline, la triptoréline.
- Les modificateurs de la réponse immune : ces substances ont pour objectif de stimuler la réponse immune anticancéreuse. Ces produits comprennent essentiellement l'interleukine 2 qui est une cytokine ayant un rôle important sur la régulation du système immunitaire, elle est produite par génie génétique, l'interleukine 2 recombinante a montré son efficacité en immunothérapie, et l'interféron α qui est une glycoprotéine produite par des cellules parasitées par un virus.

Les mécanismes d'action des médicaments anticancéreux ne sont toutefois pas toujours connus avec précision et sont parfois multiples. Ainsi, certains des médicaments cités ci-dessus peuvent être classés dans plusieurs catégories.

On entend par « cardiotoxicité » une toxicité affectant le coeur et/ou les vaisseaux. Ainsi par « effets cardiotoxiques » on entend des effets toxiques affectant le coeur et/ou les vaisseaux.

La cardiotoxicité induite par les chimiothérapies et/ou les radiations ionisantes peut revêtir plusieurs aspects cliniques, bien différents dans leur manifestation et leurs conséquences. Quatre formes cliniques ont été décrites en fonction de leur délai d'apparition par rapport à l'introduction du traitement anticancéreux :
- la cardiotoxicité aiguë : les manifestations immédiates de la cardiotoxicité des traitements anticancéreux sont principalement représentées par des tachycardies sinusales ou des anomalies de l'électrocardiogramme, comme des altérations de la repolarisation portant sur le segment ST et l'onde T. Ces manifestations sont très fréquentes et indépendantes de la dose. Elles peuvent apparaître pendant le traitement, mais surviennent plus fréquemment dans les heures qui suivent l'administration du traitement. Cette forme de toxicité, rarement grave sur le plan clinique, ne permet habituellement pas de prédire la survenue de toxicité chronique et n'est généralement pas une cause d'abandon du traitement.
- la cardiotoxicité subaiguë : cette forme de toxicité peut s'observer quelques jours ou quelques semaines après la dernière administration du traitement anticancéreux. Certaines de ces complications, comme les troubles du rythme ventriculaires (e.g., fibrillation ventriculaire), les péricardites et/ou myocardites peuvent être particulièrement graves et engager le pronostic vital. L'amélioration sans séquelle de ces manifestations est toutefois possible.
- la cardiotoxicité chronique : cette forme de toxicité chronique apparaît cliniquement le plus souvent plusieurs semaines ou plusieurs mois après la dernière cure de chimiothérapie. Elle est observée, selon les études, chez 0,4 à 23% des patients traités pour un cancer et se traduit par une insuffisance cardiaque congestive, à prédominance gauche, dont la mortalité est élevée.

L'insuffisance cardiaque est définie cliniquement comme « un état où le coeur n'est plus à même de perfuser suffisamment les organes périphériques au repos et à l'effort ». Elle peut être objectivée ou non au travers de la fraction d'éjection ventriculaire ou de la fraction d'éjection ventriculaire gauche définie comme le rapport entre le volume d'éjection systolique sur le volume diastolique.
- la cardiotoxicité tardive : le suivi à long terme des malades traités par chimiothérapie a montré, en particulier chez les enfants, qu'une forme de toxicité cardiaque pouvait être diagnostiquée plusieurs années, voire des décennies après la dernière administration d'agent(s) anticancéreux et cela, même chez des patients ayant reçu des doses cumulées plus faibles que celles classiquement rapportées comme toxiques. En effet, chez l'enfant l'expression clinique de la cardiotoxicité est moins franche que chez l'adulte, vraisemblablement du fait des réserves contractiles importantes. De ce fait, les patients jeunes peuvent rester asymptomatiques pendant des années avant qu'une insuffisance cardiaque ne se manifeste. Chez ces insuffisants cardiaques, 20% subissent une transplantation cardiaque et plus de 40% d'entre eux montrent des décompensations très tardives se traduisant par des anomalies électriques et échographiques de contractilité myocardique. Le mécanisme particulier de cette cardiotoxicité est lié au fait que le nombre de cardiomyocytes est établit de façon définitive dès l'âge de 6 mois, la croissance cardiaque se faisant ensuite par la seule hypertrophie des fibres.

Bien que les effets cardiovasculaires varient en fonction des traitements anticancéreux utilisés, ils coexistent souvent, vraisemblablement du fait de mécanismes communs. Schématiquement, ils peuvent être classés en différentes catégories de symptômes (Hong et al. 2010, Clin. Cardiol. 33, 733-737).
1/ Les dysfonctions cardiaques systoliques : on entend par « dysfonction cardiaque systolique » une anomalie de la fonction systolique conduisant à un défaut d'expulsion du sang. Elles apparaissent progressivement en raison d'une perte cellulaire du myocarde conséquence des effets cytotoxiques directs des médicaments anticancéreux tels que les anthracyclines, les anthraquinones, les anticorps monoclonaux, les inhibiteurs de tyrosine kinase, les agents alkylants, l'interféron α.
2/ L'ischémie cardiaque : on entend par « ischémie cardiaque » un défaut d'apport sanguin au coeur. L'ischémie cardiaque a été décrite chez des patients qui recevaient des antimétabolites, des intercalants ou des antibiotiques antitumoraux. Chez les patients ayant des antécédents cardiaques les évènements cardiaques peuvent même aller jusqu'à l'infarctus du myocarde. Des épisodes ischémiques sévères ont également été décrits lors d'utilisation d'agents tubulo-affins, d'inhibiteurs de facteurs de croissances. Il est possible que tous les agents antimitotiques puissent endommager les endothéliums vasculaires conduisant à des ischémies cardiaques.
3/ Les arythmies cardiaques : on entend par « arythmie cardiaque » un rythme déréglé avec des battements cardiaques irréguliers. Ces troubles du rythme peuvent résulter d'une cardiotoxicité directe mais peuvent aussi avoir une cause indirecte. Par exemple l'administration de cisplatine ou d'interleukine 2 accroit la perméabilité capillaire et induit une diminution du volume intravasculaire pouvant conduire à des arythmies ventriculaires. De nombreux agents anticancéreux allongent l'espace QT et l'association de tels médicaments peut avoir des conséquences dramatiques.
4/ Les péricardites : on entend par péricardite, une inflammation du péricarde. Elles ont été décrites chez des patients sous thérapie d'agent alkylant ou d'antimétabolite.
5/ Les complications thromboemboliques : elles sont principalement associées à l'utilisation d'inhibiteurs de facteurs de croissance, des modulateurs du récepteur des oestrogènes et de la progestérone.
6/ L'utilisation des radiations ionisantes au cours d'une radiothérapie thoracique augmente le risque de péricardites constrictives, de fibrose myocardique mais aussi de lésions valvulaires et des artères coronaires.

Les problèmes cardiovasculaires sont, de plus, exacerbés lorsque plusieurs médicaments anticancéreux sont utilisés en combinaisons et/ou associés à de la radiothérapie thoracique. De même, les patients cancéreux jeunes ou au contraire âgés, ou ceux cumulant des facteurs de risque cardiovasculaire sont particulièrement sensible. Par exemple les anthracyclines ou le 5-fluoro-uracile sont contre-indiqués chez les cancéreux ayant des antécédents cardiovasculaires. On entend par « patients cancéreux jeunes » des patients de moins de 30 ans et préférentiellement de moins de 20 ans. De même, on entend par « patients cancéreux âgés » des patients de plus de 60 ans et préférentiellement de plus de 70 ans. On entend par « souffrant d'une maladie cardiovasculaire » n'importe quelle maladie qui touche le système cardiovasculaire, on peut citer par exemple l'hypertension artérielle, l'angor, l'insuffisance cardiaque, les troubles du rythme, les maladies veineuses et/ou artérielles, le diabète. On entend par « cumulant des facteurs de risque cardiovasculaire » des patients qui présentent au moins 2 facteurs de risques cardiovasculaire, parmi tous les facteurs de risque cardiovasculaire existants, on peut citer comme exemple de facteur, l'hypertension artérielle, l'obésité, le diabète, un taux trop élevé de cholestérol, l'inactivité physique.

Certains médicaments adjuvants, comme par exemple les inhibiteurs de COX, très fréquemment utilisés pour combattre les douleurs associées aux cancers, ont eux-mêmes des effets néfastes sur le système cardiovasculaire qui s'ajoutent à ceux des traitements anticancéreux (Senkus and Janssem 2011, Cancer Treatment Reviews 37, 300-311).

Enfin, la cardiotoxicité étant généralement cumulative, un traitement anticancéreux de longue durée même à faible dose peut devenir cardiotoxique à long terme.

Globalement, les médicaments anticancéreux quel que soit leur mécanisme d'action causent des complications cardiovasculaires bien évidemment indésirables. La qualité de vie d'une proportion significative des patients aussi bien ceux en cours de traitement que ceux en rémission prolongée ou guéris de leur cancer, s'en trouve fortement diminuée. L'étendue du problème est telle qu'il fait l'objet d'une nouvelle spécialité médicale : l'onco-cardiologie (Eschenhagen et al. 2011, Eur. J. Heart Fail. 13, 1-10). A ce titre, il est nettement plus avantageux à tous les points de vue de prévenir l'apparition des problèmes/maladies cardiovasculaires provoqués par les traitements anticancéreux que d'essayer de les traiter après leur survenue.

La prévention des effets cardiotoxiques consiste à éviter leur survenue chez le patient qui ne souffre d'aucun signe/symptôme d'ordre cardiovasculaire (prévention primaire) mais consiste aussi à empêcher leur aggravation chez le patient chez qui ils sont déjà présents (prévention secondaire).

On entend par traitement des effets cardiotoxiques la disparition ou l'amélioration desdits effets.

Diverses stratégies sont mises en oeuvre pour réduire l'incidence et/ou la gravité des effets secondaires des médicaments anticancéreux, et donc des effets cardiovasculaires qui en font partie. On peut, par exemple, augmenter la concentration du/des principe(s) actif(s) au niveau de la tumeur et/ou de l'organe cible par des moyens physiques, physicochimiques, galéniques ou un adressage sélectif. Une autre approche, plus spécifiquement tournée vers la protection du système cardiovasculaire utilise le dexrazoxane, seul médicament approuvé dans les cardiotoxicités induites par la doxorubicine (Ewer et al., 2010, Nature Reviews. Cardiology, 564-575; Singal et al., 1998, New England Journal of Medicine, 900-905). Toutefois son utilisation est de plus en plus controversée. D'autres médicaments tels que l'érythropoïétine, la thrombopoïétine et l'iloprost se sont avérés potentiellement utiles dans la prévention de la cardiotoxicité due aux anthracyclines dans des modèles animaux mais n'ont pas été évalués en clinique humaine (Li et al. 2006, Circulation 113, 535-543 ; Li et al. 2006, Circulation 113, 2211-2220).

Selon cette dernière approche, il est clair que l'agent protecteur ne doit agir qu'au niveau des cellules du système cardiovasculaire et ne doit en aucun cas s'opposer à l'action antitumorale du traitement. La sélectivité d'action de l'agent protecteur est donc un critère essentiel qui n'est pas satisfait dans le cas des agents existants tels que le dexrazoxane et autres chélateurs de fer et/ou antioxydants dont les mécanismes d'action tendent à interférer avec celui des traitements anticancéreux et nuire ainsi à leur efficacité sur le plan cancérologique.

Il existe donc un besoin dans l'état de la technique pour l'identification de substances capables de protéger sélectivement le système cardiovasculaire des effets toxiques des médicaments anticancéreux et/ou des radiations ionisantes.

De façon inattendue, les inventeurs ont découvert que la 3-(R)-[3-(2-méthoxyphénylthio)-2-(S)-méthyl-propyl]amino-3,4-dihydro-2H-1,5-benzoxathiépine, ou l'un de ses sels pharmaceutiquement acceptables, est capable de protéger sélectivement le système cardiovasculaire des effets toxiques des médicaments anticancéreux et/ou des radiations ionisantes. A ce titre, la 3-(R)-[3-(2-méthoxyphénylthio)-2-(S)-méthyl-propyl] amino-3,4-dihydro-2H-1,5-benzoxathiépine répond donc à un besoin médical important.

Dans la présente invention, le terme « pharmaceutiquement acceptable » se réfère à des entités moléculaires et des compositions qui ne produisent aucun effet adverse, allergique ou autre réaction indésirable quand elles ont administrées à un humain. Quand utilisé ici, le terme « excipient pharmaceutiquement acceptable » inclut tout diluant, adjuvant ou excipient, tels que des agents préservatifs, des agents de remplissage, des agents désintégrant, mouillant, émulsifiant, dispersant, antibactérien ou antifongique, ou bien encore des agents qui permettraient de retarder l'absorption et la résorption intestinale et digestive. L'utilisation de ces milieux ou vecteurs est bien connu de l'homme du métier.

On désigne par « sels pharmaceutiquement acceptables » d'un composé les sels définis ici et qui possèdent l'activité pharmacologique du composé parent. De tels sels comprennent : les sels d'addition d'acide formés avec des acides minéraux tels que l'acide chlorhydrique, l'acide bromhydrique, l'acide sulfurique, l'acide nitrique, l'acide phosphorique et similaires ou formés avec des acides organiques tels que l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzoyl-L-tartrique, l'acide tartrique, l'acide p-toluènesulfonique, l'acide triméthylacétique, l'acide trifluoroacétique et similaires.

Les sels pharmaceutiquement acceptables comprennent également les formes d'addition de solvants (solvates) ou les formes cristallines (polymorphes) tels que définis ici, du même sel d'addition d'acide.

La 3-(R)-[3-(2-méthoxyphénylthio)-2-(S)-méthyl-propyl]amino-3,4-dihydro-2H-1,5-benzoxathiépine est un bloqueur sélectif du courant sodique lent produit par le canal voltage dépendant Nav1.5, abrégé ici courant sodique Nav1.5 lent. Il est important de noter que ce composé n'est pas cyctotoxique et n'est pas doté de propriétés antioxydantes.

Le blocage du courant sodique Nav1.5 lent ne fait pas partie des mécanismes mis en jeu par les médicaments anticancéreux et les radiations ionisantes pour détruire et/ou limiter la croissance des tumeurs.

Le canal Nav1.5 est essentiellement distribué au niveau des cellules musculaire du coeur et des vaisseaux sanguins. De plus, le courant sodique Nav1.5 lent n'a pas de rôle dans le fonctionnement normal des cellules cardiaques et vasculaires. A ce titre, les inventeurs ont déjà montré que la 3-(R)-[3-(2-méthoxyphénylthio)-2-(S)-méthyl-propyl]amino-3,4-dihydro-2H-1,5-benzoxathiépine même aux fortes concentrations n'affectent pas le fonctionnement normal du système cardiovasculaire.

Compte tenu de son mode d'action particulier, les inventeurs ont montré de façon surprenante, que la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables prévient ou atténue fortement la cardiotoxicité induite par la doxorubicine sans pour autant en diminuer la toxicité vis à vis des cellules cancéreuses.

Sur la base de cette observation, les inventeurs considèrent que le courant sodique Nav1.5 lent est produit ou amplifié lorsque les cellules cardiaques et/ou vasculaires sont soumises à l'action de la doxorubicine ou plus généralement d'un ou plusieurs médicaments anticancéreux et/ou des radiations ionisantes.

Les inventeurs considèrent aussi que le courant sodique Nav1.5 lent produit ou amplifié dans les cardiomyocytes en réponse à l'exposition aux médicaments anticancéreux et/ou des radiations ionisantes est responsable de leur cardiotoxicité.

Ainsi, le blocage sélectif du courant sodique Nav1.5 lent permet de prévenir la cardiotoxicité des médicaments anticancéreux et/ou des radiations ionisantes sans en diminuer l'efficacité sur le plan cancérologique.

De ce fait, la 3-(R)-[3-(2-méthoxyphénylthio)-2-(S)-méthyl-propyl]amino-3,4-dihydro-2H-1,5-benzoxathiépine, ainsi que ses sels pharmaceutiquement acceptables, possèdent la sélectivité d'action requise et indispensable pour être utile en tant qu'agent protecteur du système cardiovasculaire, en particulier lors de traitements anticancéreux.

La présente invention concerne l'utilisation de la 3-(R)-[3-(2-méthoxyphénylthio)-2-(S)-méthyl-propyl]amino-3,4-dihydro-2H-1,5-benzoxathiépine, ou l'un de ses sels pharmaceutiquement acceptables comme médicament pour la prévention et/ou le traitement des effets cardiotoxiques dus aux médicaments anticancéreux et/ou aux radiations ionisantes.

La présente invention concerne l'utilisation de la 3-(R)-[3-(2-méthoxyphénylthio)-2-(S)-méthyl-propyl]amino-3,4-dihydro-2H-1,5-benzoxathiépine, ou l'un de ses sels pharmaceutiquement acceptables comme médicament pour la prévention et/ou le traitement des effets cardiotoxiques dus aux médicaments anticancéreux choisis parmi les agents alkylants, les antimétabolites, les modificateurs de l'ADN, les antibiotiques antitumoraux, les dérivés tubulo-affins, les inhibiteurs de facteurs de croissances, les modificateurs de la réponse immune et hormonale et/ou les associations possibles de ces différentes catégories de médicaments, en particulier la doxorubicine.

La présente invention concerne aussi l'utilisation de la 3-(R)-[3-(2-méthoxyphénylthio)-2-(S)-méthyl-propyl]amino-3,4-dihydro-2H-1,5-benzoxathiépine, ou l'un de ses sels pharmaceutiquement acceptables comme médicament pour la prévention et/ou le traitement des effets cardiotoxiques dus aux radiations ionisantes et/ou à l'association des radiations ionisantes avec les différentes classes de médicaments anticancéreux.

Selon la présente invention les effets cardiotoxiques dus aux médicaments anticancéreux et/ou aux radiations ionisantes se traduisent préférentiellement soit par des troubles du rythme, une ischémie cardiaque, des dysfonctions cardiaques systoliques, une péricardite, une trombophilie, ou bien encore par une insuffisance cardiaque.

La présente invention concerne des patients cancéreux traités qui ont une fraction d'éjection ventriculaire ou une fraction d'éjection ventriculaire gauche inférieure à la normale à l'initiation du traitement anticancéreux ou ceux qui ont une fraction d'éjection ventriculaire qui diminue au cours de ce traitement.

La présente invention concerne également des patients cancéreux traités qui sont soit jeunes, ou âgés, qui souffrent d'une maladie cardiovasculaire ou bien encore qui cumulent des facteurs de risque cardiovasculaire.

La 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables, peut être administré simultanément avec les traitements anticancéreux ou de façon séparée ou décalée. Il peut également être utilisé pendant toute la durée ou sur une durée plus courte ou plus longue que celle du traitement anticancéreux.

La présente invention a aussi pour objet l'utilisation de la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables pour la fabrication d'un médicament destiné à la prévention ou au traitement de la cardiotoxicité qui apparait pendant ou après un ou plusieurs traitement(s) anticancéreux.

La présente invention concerne en outre une composition pharmaceutique comprenant la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables comme principe actif et au moins un excipient pharmaceutiquement acceptable.

Les compositions pharmaceutiques selon la présente invention peuvent être formulées pour l'administration à l'être humain. Les compositions selon l'invention peuvent être administrées par voie orale, sublinguale, sous-cutanée, intramusculaire, intraveineuse, transdermique, locale ou rectale ou bien encore intra-nasale. Dans ce cas le principe actif peut être administré sous formes unitaires d'administration, en mélange avec des supports pharmaceutiques classiques, aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée ou transdermique, topique, intramusculaire, intraveineuse, intra-nasale ou intraoculaires, les formes d'administration rectale.

Lorsque l'on prépare une composition solide sous forme de comprimés, on mélange le ingrédient actif principal avec un véhicule pharmaceutique tel que la gélatine, l'amidon, le lactose, le stéarate de magnésium, le talc, la gomme arabique, la silice ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillant, ou des agents de mise en suspension, de même qu'avec des correcteurs du goût ou des édulcorants.

Pour une administration rectale, on recourt à des suppositoires qui sont préparés avec des liants fondant à la température rectale, par exemple du beurre de cacao ou des polyéthylènes glycols.

Pour une administration parentérale (intraveineuse, intramusculaire, intradermique, sous-cutanée), intra-nasale ou intraoculaire, on utilise des suspensions aqueuses, des solutions salines isotoniques ou des solutions stériles et injectables qui contiennent des agents de dispersion et/ou des agents mouillants pharmacologiquement compatibles.

Le principe actif peut également être formulé sous forme de microcapsules, éventuellement avec un ou plusieurs supports additifs.

Avantageusement, la composition pharmaceutique selon la présente invention est destinée à une administration par voie orale ou intraveineuse.

La composition pharmaceutique selon la présente invention peut être administrée simultanément avec les médicaments anticancéreux ou de façon séparée ou décalée. Il peut également être utilisé pendant toute la durée ou sur une durée plus courte ou plus longue que celle du traitement anticancéreux.

La composition pharmaceutique selon la présente invention peut comprendre d'autres principes actifs conduisant à un effet complémentaire ou éventuellement synergique.

Les dosages de la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables dans les compositions de l'invention peuvent être ajustés afin d'obtenir une quantité de substance qui est efficace pour obtenir la réponse thérapeutique désirée pour une composition particulière à la méthode d'administration. La dose efficace du composé de l'invention varie en fonction de nombreux paramètres tels que, par exemple, la voie d'administration choisie, le poids, l'âge, le sexe, la nature de la pathologie, le type du ou des traitements anticancéreux administrés et la sensibilité de l'individu à traiter. En conséquence, la posologie optimale devra être déterminée par le spécialiste en la matière en fonction des paramètres qu'il juge pertinents. Bien que les doses efficaces puissent varier dans de larges proportions, les doses journalières pourraient s'échelonner entre 1 mg et 1000 mg par 24 heures, et préférentiellement entre 1 et 200 mg, pour un adulte d'un poids moyen de 70 kg, en une ou plusieurs prises.

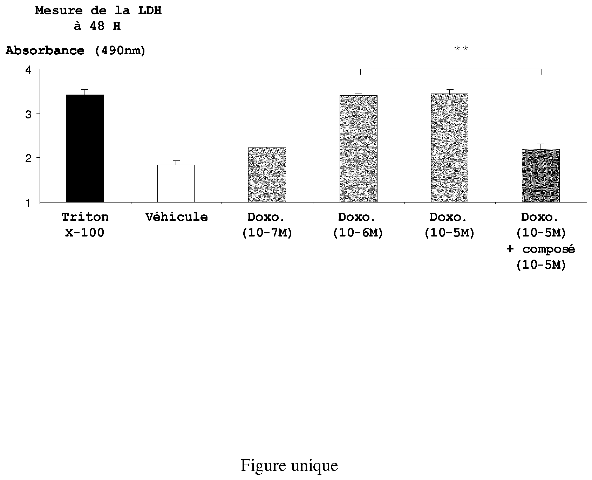

L'invention sera mieux comprise en référence à l'exemple et à la figure qui suivent.

Il est montré selon l'invention que la 3-(R)-[3-(2-méthoxyphénylthio)-2-(S)-méthyl-propyl]amino-3,4-dihydro-2H-1,5-benzoxathiépine possède la propriété remarquable et inattendue de préserver la viabilité des cardiomyocytes exposés à des concentrations toxiques d'un médicament anticancéreux : la doxorubicine. Dans la présente invention, la doxorubicine a été choisie à titre purement illustratif sur la base de sa cardiotoxiocité reconnue. Il est entendu que l'utilité de la 3-(R)-[3-(2-méthoxyphénylthio)-2-(S)-méthyl-propyl]amino-3,4-dihydro-2H-1,5-benzoxathiépine, ou l'un de ses sels pharmaceutiquement acceptables, s'étend aux autres médicaments ou combinaisons de médicaments anticancéreux ainsi qu'aux radiations ionisantes.

Les effets de la 3-(R)-[3-(2-méthoxyphénylthio)-2-(S)-méthyl-propyl]amino-3,4-dihydro-2H-1,5-benzoxathiépine ont été évalués dans le modèle cellulaire suivant : des cardiomyocytes de rat sont mis en culture soit en présence soit en l'absence de doxorubicine. Après 48 heures, la souffrance cellulaire est quantifiée au moyen du dosage de la concentration de la lactatedeshydrogénase (LDH); la LDH est un marqueur de la glycolyse anaérobie.

On constate que la doxorubicine à la concentration de 1 et 10 µM affecte la viabilité des cardiomyocytes de façon significative et comparable à celle d'un agent toxique choisi comme de référence : le Triton X-100 (voir Figure annexée). On constate par ailleurs que la 3-(R)-[3-(2-méthoxyphénylthio)-2-(S)-méthyl-propyl]amino-3,4-dihydro-2H-1,5-benzoxathiépine, à la concentration de 10 µM réduit significativement les effets toxiques de la doxorubicine et préserve ainsi la viabilité des cardiomyocytes.

La doxorubicine n'est pas connue pour interagir directement avec le canal sodique Nav1.5. D'après les résultats de l'expérience il est néanmoins clair que ledit canal joue un rôle déterminant dans la cardiotoxicité de la doxorubicine. Sur la base de cette observation surprenante, et sans préjuger du détail des mécanismes de cytotoxicité mis en jeu par la doxorubicine et plus généralement par les traitements anticancéreux, les inventeurs avancent que le courant sodique Nav1.5 lent produit par le cardiomyocyte en réponse à son exposition à la doxorubicine est nécessaire et suffisant pour rendre compte de sa toxicité et plus généralement de celle des traitements anticancéreux. En d'autres termes, dans les cellules cardiaques et vasculaires, l'expression ou l'amplification du courant sodique lent produit par le canal Nav1.5 représente une réponse commune aux traitements anticancéreux et responsable de la survenue des cardiotoxicités.

Il est important de noter que dans les lignées cellulaires cancéreuses sensibles à la doxorubicine, le mélange doxorubicine/3-(R)-[3-(2-méthoxyphénylthio)-2-(S)-méthyl-propyl]amino-3,4-dihydro-2H-1,5-benzoxathiépine est au moins aussi cytotoxique vis-à-vis des cellules tumorales que la doxorubicine seule. La 3-(R)-[3-(2-méthoxyphénylthio)-2-(S)-méthyl-propyl]amino-3,4-dihydro-2H-1,5-benzoxathiépine n'interfère donc pas avec les mécanismes responsables de la cytotoxicité de la doxorubicine vis-à-vis des cellules cancéreuses.

## Revendications

1. La 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables pour son utilisation comme médicament dans la prévention et/ou le traitement des effets cardiotoxiques dus aux médicaments anticancéreux et/ou aux radiations ionisantes.

2. La 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables pour son utilisation selon la revendication 1, **caractérisée en ce que** les médicaments anticancéreux sont choisis parmi les agents alkylants, les antimétabolites, les agents modificateurs de l'ADN, les antibiotiques antitumoraux, les dérivés tubulo-affins, les inhibiteurs de facteurs de croissances, les modificateurs de la réponse immune ou hormonale et/ou les associations possibles de ces différentes catégories de médicaments.

3. La 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables pour son utilisation selon la revendication 1, **caractérisée en ce que** le médicament anticancéreux est la doxorubicine.

4. La 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables pour son utilisation selon l'une des revendications 1 et 2, **caractérisée en ce que** les effets cardiotoxiques se traduisent par des troubles du rythme, une ischémie cardiaque, des dysfonctions cardiaques systoliques, une péricardite, une thrombophilie ou une insuffisance cardiaque.

5. La 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables pour son utilisation selon l'une quelconque des revendications 1 à 4 chez des patients cancéreux traités, jeunes ou âgés, et souffrant d'une maladie cardiovasculaire et/ou cumulant des facteurs de risque cardiovasculaire.

6. La 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables pour son utilisation selon l'une des revendications 1 à 5 chez des patients cancéreux traités ayant une fraction d'éjection ventriculaire ou une fraction d'éjection ventriculaire gauche inférieure à la normale à l'initiation du traitement anticancéreux ou ceux qui ont une fraction d'éjection ventriculaire qui diminue au cours de ce traitement.

7. Composition pharmaceutique **caractérisée en ce qu'**elle contient comme principe actif la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables et au moins un excipient pharmaceutiquement acceptable, pour son utilisation comme médicament dans la prévention et/ou le traitement des effets cardiotoxiques dus aux médicaments anticancéreux et/ou aux radiations ionisantes.

8. Composition pharmaceutique pour son utilisation selon la revendication 7, **caractérisée en ce que** les médicaments anticancéreux sont choisis parmi les agents alkylants, les antimétabolites, les agents modificateurs de l'ADN, les antibiotiques antitumoraux, les dérivés tubulo-affins, les inhibiteurs de facteurs de croissances, les modificateurs de la réponse immune et hormonale et/ou les associations possibles de ces différentes catégories de médicaments.

9. Composition pharmaceutique pour son utilisation selon la revendication 7, **caractérisée en ce que** le médicament anticancéreux est la doxorubicine.

10. Composition pharmaceutique pour son utilisation selon l'une des revendications 7 à 9, **caractérisée en ce que** les effets cardiotoxiques se traduisent par des troubles du rythme, une ischémie cardiaque, des dysfonctions cardiaques systoliques, une péricardite, une thrombophilie ou une insuffisance cardiaque.

11. Composition pharmaceutique pour son utilisation selon l'une quelconque des revendications 7 à 10, pour son utilisation chez des patients cancéreux traités ayant une fraction d'éjection ventriculaire ou une fraction d'éjection ventriculaire gauche inférieure à la normale à l'initiation du traitement anticancéreux ou ceux qui ont une fraction d'éjection ventriculaire qui diminue au cours de ce traitement.

12. Composition pharmaceutique pour son utilisation selon l'une quelconque des revendications 7 à 11, pour son administration par voie orale ou intraveineuse.

13. Composition pharmaceutique pour son utilisation selon l'une quelconque des revendications 7 à 12, pour son administration simultanément avec des médicaments anticancéreux.

14. Composition pharmaceutique pour son utilisation selon l'une quelconque des revendications 7 à 13, pour son administration de façon séparée ou décalée avec des médicaments anticancéreux.

15. Composition pharmaceutique pour son utilisation selon l'une quelconque des revendications 7 à 14, **caractérisée en ce qu'**elle est présentée sous la forme d'une unité de dosage quotidienne de la 3-(R)-[3-(2-méthoxyphénylthio)-2-(S)-méthyl-propyl]amino-3,4-dihydro-2H-1,5-benzoxathiépine ou l'un de ses sels pharmaceutiquement acceptables comprise entre 1 et 1000 mg.

## Patentansprüche

1. 3-(R)-[3-(2-methoxyphenylthio)-2-(S)-methyl-propyl]amino-3,4-dihydro-2H-1,5-benzoxathiepin oder eines seiner pharmazeutisch akzeptablen Salze für seine Verwendung als Arzneimittel zur Prävention und/oder Behandlung von durch Anti-Krebs-Arzneimittel und/oder ionisierende Strahlungen verursachten kardiotoxischen Wirkungen.

2. 3-(R)-[3-(2-methoxyphenylthio)-2-(S)-methyl-propyl]amino-3,4-dihydro-2H-1,5-benzoxathiepin oder eines seiner pharmazeutisch akzeptablen Salze für seine Verwendung nach Anspruch 1, **dadurch gekennzeichnet, dass** die Anti-Krebs-Arzneimittel aus den Alkylierungsmitteln, den Antimetaboliten, den DNA-Modifizierungsmitteln, den antitumoralen Antibiotika, den tubulo-affinen Derivaten, den Wachstumsfaktorinhibitoren, den Modifikatoren der Immun- oder Hormonantwort und/oder den möglichen Kombinationen dieser verschiedenen Arzneimittelkategorien ausgewählt sind.

3. 3-(R)-[3-(2-methoxyphenylthio)-2-(S)-methyl-propyl]amino-3,4-dihydro-2H-1,5-benzoxathiepin oder eines seiner pharmazeutisch akzeptablen Salze für seine Verwendung nach Anspruch 1, **dadurch gekennzeichnet, dass** das Anti-Krebs-Arzneimittel Doxorubicin ist.

4. 3-(R)-[3-(2-methoxyphenylthio)-2-(S)-methyl-propyl]amino-3,4-dihydro-2H-1,5-benzoxathiepin oder eines seiner pharmazeutisch akzeptablen Salze für seine Verwendung nach einem der Ansprüche 1 und 2, **dadurch gekennzeichnet, dass** sich die kardiotoxischen Wirkungen anhand von Rhythmusstörungen, einer Herz-Ischämie, von systolischen Herz-Dysfunktionen, einer Pericarditis, einer Thrombophilie oder einer Herzinsuffizienz manifestieren.

5. 3-(R)-[3-(2-methoxyphenylthio)-2-(S)-methyl-propyl]amino-3,4-dihydro-2H-1,5-benzoxathiepin oder eines seiner pharmazeutisch akzeptablen Salze für seine Verwendung nach einem der Ansprüche 1 bis 4 bei behandelten jungen oder alten Krebspatienten, die an einer kardiovaskulären Erkrankung leiden und/oder kardiovaskuläre Risikofaktoren kumulieren.

6. 3-(R)-[3-(2-methoxyphenylthio)-2-(S)-methyl-propyl]amino-3,4-dihydro-2H-1,5-benzoxathiepin oder eines seiner pharmazeutisch akzeptablen Salze für seine Verwendung nach einem der Ansprüche 1 bis 5 bei behandelten Krebspatienten mit einer ventrikulären Ejektionsfraktion oder einer ventrikulären Ejektionsfraktion links unterhalb des Normbereichs zu Beginn der Anti-Krebs-Behandlung oder solchen, die eine ventrikuläre Ejektionsfraktion haben, die sich im Verlauf dieser Behandlung verringert.

7. Pharmazeutische Zusammensetzung, **dadurch gekennzeichnet, dass** sie als Wirkstoff 3-(R)-[3-(2-methoxyphenylthio)-2-(S)-methyl-propyl]amino-3,4-dihydro-2H-1,5-benzoxathiepin oder eines seiner pharmazeutisch akzeptablen Salze und mindestens einen pharmazeutisch akzeptablen Hilfsstoff für ihre Verwendung als Arzneimittel zur Prävention und/oder Behandlung von durch Anti-Krebs-Arzneimittel und/oder ionisierende Strahlungen verursachten kardiotoxischen Wirkungen enthält.

8. Pharmazeutische Zusammensetzung für ihre Verwendung nach Anspruch 7, **dadurch gekennzeichnet, dass** die Anti-Krebs-Arzneimittel aus den Alkylierungsmitteln, den Antimetaboliten, den DNA-Modifizierungsmitteln, den antitumoralen Antibiotika, den tubulo-affinen Derivaten, den Wachstumsfaktorinhibitoren, den Modifikatoren der Immun- oder Hormonantwort und/oder den möglichen Kombinationen dieser verschiedenen Arzneimittelkategorien ausgewählt sind.

9. Pharmazeutische Zusammensetzung für ihre Verwendung nach Anspruch 7, **dadurch gekennzeichnet, dass** das Anti-Krebs-Arzneimittel Doxorubicin ist.

10. Pharmazeutische Zusammensetzung für ihre Verwendung nach einem der Ansprüche 7 bis 9, **dadurch gekennzeichnet, dass** sich die kardiotoxischen Wirkungen anhand von Rhythmusstörungen, einer Herz-Ischämie, von systolischen Herz-Dysfunktionen, einer Pericarditis, einer Thrombophilie oder einer Herzinsuffizienz manifestieren.

11. Pharmazeutische Zusammensetzung für ihre Verwendung nach einem der Ansprüche 7 bis 10 bei behandelten Krebspatienten mit einer ventrikulären Ejektionsfraktion oder einer ventrikulären Ejektionsfraktion links unterhalb des Normbereichs zu Beginn der Anti-Krebs-Behandlung oder solchen, die eine ventrikuläre Ejektionsfraktion haben, die sich im Verlauf dieser Behandlung verringert.

12. Pharmazeutische Zusammensetzung für ihre Verwendung nach einem der Ansprüche 7 bis 11 für ihre Verabreichung auf dem oralen oder intravenösen Weg.

13. Pharmazeutische Zusammensetzung für ihre Verwendung nach einem der Ansprüche 7 bis 12 für ihre gleichzeitige Verabreichung mit Anti-Krebs-Arzneimitteln.

14. Pharmazeutische Zusammensetzung für ihre Verwendung nach einem der Ansprüche 7 bis 13 für ihre separate oder versetzte Verabreichung mit Anti-Krebs-Arzneimitteln.

15. Pharmazeutische Zusammensetzung für ihre Verwendung nach einem der Ansprüche 7 bis 14, **dadurch gekennzeichnet, dass** sie in Form einer täglichen Dosierungseinheit von 3-(R)-[3-(2-methoxyphenylthio)-2-(S)-methyl-propyl]amino-3,4-dihydro-2H-1,5-benzoxathiepin oder eines seiner pharmazeutisch akzeptablen Salze zwischen 1 und 1000 mg vorliegt.

## Claims

1. 3-(R)-[3-(2-methoxyphenylthio)-2-(S)-methylpropyl]amino-3,4-dihydro-2H-1,5-benzoxathiepine or one of its pharmaceutically acceptable salts for use as drug in the prevention and/or treatment of the cardiotoxic effects due to anticancer drugs and/or ionising radiation.

2. 3-(R)-[3-(2-methoxyphenylthio)-2-(S)-methylpropyl]amino-3,4-dihydro-2H-1,5-benzoxathiepine or one of its pharmaceutically acceptable salts for use according to claim 1, **characterized in that** the anticancer drugs are chosen from among alkylating agents, antimetabolites, DNA modifying agents, antitumour antibiotics, mitotic spindle poison derivatives, growth factor inhibitors, modifiers of immune or hormonal response and/or possible associations of these different categories of drugs.

3. 3-(R)-[3-(2-methoxyphenylthio)-2-(S)-methyl-propyl]amino-3,4-dihydro-2H-1,5-benzoxathiepine or one of its pharmaceutically acceptable salts for use according to claim 1, **characterized in that** the anticancer drug is doxorubicin.

4. 3-(R)-[3-(2-methoxyphenylthio)-2-(S)-methylpropyl]amino-3,4-dihydro-2H-1,5-benzoxathiepine or one of its pharmaceutically acceptable salts for use according to one of claims 1 and 2, **characterized in that** the cardiotoxic effects translate as rhythm disorders, cardiac ischemia, systolic cardiac dysfunction, pericarditis, thrombophilia or heart failure.

5. 3-(R)-[3-(2-methoxyphenylthio)-2-(S)-methylpropyl]amino-3,4-dihydro-2H-1,5-benzoxathiepine or one of its pharmaceutically acceptable salts for use according to any one of claims 1 to 4 in treated cancer patients, young or elderly and suffering from cardiovascular disease and/or accumulating cardiovascular risk factors.

6. 3-(R)-[3-(2-methoxyphenylthio)-2-(S)-methylpropyl]amino-3,4-dihydro-2H-1,5-benzoxathiepine or one of its pharmaceutically acceptable salts for use according to one of claims 1 to 5 in treated cancer patients having a ventricular ejection fraction or left ventricular ejection fraction lower than normal on initiation of anticancer treatment, or those having a ventricular ejection fraction which decreases throughout the course of this treatment.

7. A pharmaceutical composition **characterized in that** it contains as active ingredient 3-(R)-[3-(2-methoxyphenylthio)-2-(S)-methyl-propyl]amino-3,4-dihydro-2H-1,5-benzoxathiepine or one of its pharmaceutically acceptable salts and at least one pharmaceutically acceptable excipient for use as drug in the prevention and/or treatment of cardiotoxic effects due to anticancer drugs and/or to ionising radiation.

8. The pharmaceutical composition for use according to claim 7, **characterized in that** the anticancer drugs are chosen from among alkylating agents, antimetabolites, DNA modifying agents, antitumour antibiotics, mitotic spindle poison derivatives, growth factor inhibitors, modifiers of immune and hormonal response and/or possible associations of these different categories of drugs.

9. The pharmaceutical composition for use according to claim 7, **characterized in that** the anticancer drug is doxorubicin.

10. The pharmaceutical composition for use according to one of claims 7 to 9, **characterized in that** the cardiotoxic effects translate as rhythm disorders, cardiac ischemia, systolic cardiac dysfunction, pericarditis, thrombophilia or heart failure.

11. The pharmaceutical composition for use according to any one of claims 7 to 10, for use in treated cancer patients having a ventricular ejection fraction or left ventricular ejection fraction lower than normal on initiation of the anticancer treatment, or those having a ventricular ejection fraction which decreases throughout the course of this treatment.

12. The pharmaceutical composition for use according to any one of claims 7 to 11 for administration via oral or intravenous route.

13. The pharmaceutical composition for use according to any one of claims 7 to 12 for administration thereof simultaneously with the anticancer drugs.

14. The pharmaceutical composition for use according to any one of claims 7 to 13 for separate or sequential administration thereof with the anticancer drugs.

15. The pharmaceutical composition for use according to any one of claims 7 to 14, **characterized in that** it is in the form of a daily dosage unit of 3-(R)-[3-(2-methoxyphenylthio)-2-(S)-methyl-propyl]amino-3,4-dihydro-2H-1,5-benzoxathiepine or one of its pharmaceutically acceptable salts, of between 1 and 1000 mg.
